# NEUE EUROPÄISCHE PATENTSCHRIFT

(11) **EP 2 966 158 B2**
(45) Veröffentlichungstag und Bekanntmachung des Hinweises auf die Entscheidung über den Einspruch: **13.03.2024**
(45) Hinweis auf die Patenterteilung: 22.11.2017
(21) Anmeldenummer: 14176002.5
(22) Anmeldetag: 07.07.2014
(51) Int. Cl.: C11B 9/00, A61Q 13/00, A61L 9/01, C11D 3/50, A61L 2/00, A61L 9/00

(54) **Isomerenmischungen von ungesättigten makrocyclischen Moschusverbindungen**
Isomers mixtures of unsaturated macrocyclic musk compounds
Mélanges d'isomères de composés musqués macrocyliques insaturés

(43) Veröffentlichungstag der Anmeldung: 13.01.2016
(73) Patentinhaber: Symrise AG, 37603 Holzminden (DE)
(72) Erfinder: Hölscher, Bernd, 37620 Halle (DE); Wiedmann, Wilhelm, 37639 Bevern (DE); Siegel, Sven, 37671 Höxter (DE); Kulke, Torsten, 37671 Höxter-Lüchtringen (DE)
(74) Vertreter: Eisenführ Speiser

(56) Entgegenhaltungen:
- EP-A1- 2 662 098
- EP-A1- 2 662 098
- EP-A2- 0 818 452
- WO-A1-03/037841
- WO-A1-2005/063670
- US-A- 4 568 470
- A. Furstner et al., J. Org. Chem., 1996. 61,3942-3943

## Beschreibung

Die vorliegende Erfindung betrifft primär neue Mischungen, enthaltend oder bestehend aus einer ersten Verbindung (**E**) und einer zweiten Verbindung (**Z**), wobei die Verbindungen (**E**) und (**Z**) Verbindungen der Formel (I) mit identischer Konstitutionsformel sind 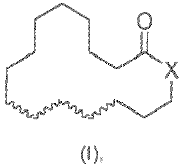 in Anspruch 1 definiert. Die vorliegende Erfindung betrifft weiterhin parfümierte Produkte enthaltend eine erfindungsgemäße Mischung (wie hierin beschrieben), Verfahren zur Herstellung erfindungsgemäßer parfümierter Produkte sowie bevorzugte Verwendungen erfindungsgemäßer Mischungen, insbesondere (a) zum Maskieren oder Vermindern des bzw. eines oder mehrerer unangenehmer Geruchseindrücke eines oder mehrerer unangenehm riechender Stoffe, und/oder (b) zum Verstärken des bzw. eines oder mehrerer angenehmer Geruchseindrücke eines oder mehrerer angenehm riechender Stoffe.

Weitere Aspekte und bevorzugte Ausgestaltungen der vorliegenden Erfindung ergeben sich aus der nachfolgenden Beschreibung, den Beispielen sowie insbesondere den beigefügten Patentansprüchen.

Grundsätzlich besteht seitens der Parfümindustrie ein ständiger Bedarf, angenehme geruchliche Aspekte von Riechstoffen zu betonen (hervorzuheben / zu verstärken) und unangenehme geruchliche Aspekte zu maskieren oder zu vermindern. Insbesondere die hierin beschriebenen mit erfindungsgemäß zu verwendenden Verbindungen der Formel (I) vorteilhafterweise bevorzugt zu kombinierende Riechstoffe spielen in der Parfümerie eine wichtige Rolle. Dabei ist es gewünscht, einerseits deren natürliche Frische und/oder Ausstrahlung besonders hervorzuheben und andererseits neue Effekte zu entwickeln. EP 2 662 098 A1 und US 4,568,470 beschreiben die Verwendung von Makroliden und makrozyklischen Ketonen zur Duftverstärkung bzw. zur Verminderung unangenehmer Dufteindrücke. Es war daher primäre Aufgabe der vorliegenden Erfindung, alternative oder vorzugsweise verbesserte Substanzen (Stoffe oder Stoffmischungen) zur Veränderung bzw. Beeinflussung geruchlicher Aspekte bereitzustellen, insbesondere um in Kombination mit Aldehyden und Alkoholen positive Effekte anzugeben.

Diese Substanzen sollten dabei vorzugsweise eine, mehrere oder vorzugsweise sämtliche der folgenden Anforderungen erfüllen:
- leichte Zugänglichkeit,
- hohe Wirksamkeit in geringer Konzentration, vorzugsweise bei in geringen Konzentrationen nicht oder kaum wahrnehmbarem Eigengeruch,
- weitgehende oder vollständige Farblosigkeit,
- hohe Stabilität in diversen Mischungen bzw. Zubereitungen, wobei insbesondere keine Verfärbung und/oder Separation und/oder Trübung auftreten soll,
- inertes Verhalten,
- keine toxische und/oder allergene Wirkung gegenüber dem Menschen.

Des Weiteren sollten mit der vorliegenden Erfindung insbesondere neue, vorteilhafte Riechstoffmischungen, v.a. Parfümöle, angegeben werden, welche solche Substanzen enthalten. Solche Riechstoffmischungen sollten vorzugsweise dafür geeignet sein, bestimmte Produkte zu beduften beziehungsweise zu parfümieren.

Weiterhin sollten demensprechend parfümierte Produkte sowie Verfahren zur Herstellung solcher Produkte angegeben werden.

Weitere Aufgabenstellungen, die der vorliegenden Erfindung zugrundeliegen, ergeben sich aus den nachfolgenden Ausführungen und den beigefügten Patentansprüchen.

Die primäre Aufgabe der vorliegenden Erfindung wird gelöst durch eine Mischung wie in Anspruch 1 definiert. 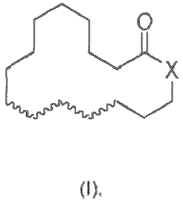 Überraschenderweise sind die neuen Mischungen (wie hierin beschrieben) sehr gut geeignet, um positive Einflüsse auf (andere) Riechstoffe zu bewirken, insbesondere auf solche Riechstoffe wie hierin beschrieben, v.a. Aldehyde und Alkohole.

Die erfindungsgemäß zu verwendenden Verbindungen der Formel (I) bzw. die hierin beschriebenen Mischungen sind weiterhin vorteilhafterweise leicht zugänglich bzw. herstellbar. Verfahren zur Herstellung einer erfindungsgemäßen Mischung sind weiter unten beschrieben.

Weiterhin weisen die erfindungsgemäß zu verwendenden Verbindungen bzw. die hierin beschriebenen erfindungsgemäßen Mischungen bereits in geringer Konzentration eine hohe Wirksamkeit auf, insbesondere bei Konzentrationen, bei denen die Verbindungen der Formel (I) keinen oder zumindest lediglich einen kaum wahrnehmbarem Eigengeruch aufweisen, sie sind vorteilhafterweise weitgehend oder vollständig farblos, besitzen eine hohe Stabilität in unterschiedlichen Mischungen bzw. Zubereitungen und weisen keine toxische und/oder allergene Wirkung gegenüber dem Menschen auf.

Verbindungen der Formel (I) bzw. die hierin beschriebenen erfindungsgemäßen Mischungen haben zudem den Vorteil, dass sie bei ihrer Verwendung, insbesondere einer erfindungsgemäßen Verwendung, mit unterschiedlichen Riechstoffen und Parfümölen bzw. üblichen Bestandteilen eines Parfümöls kombiniert werden können, um Produkte mit einer beliebigen Duftrichtung zu parfümieren. Folglich lässt sich durch die vorliegende Erfindung den Verbrauchern gegenüber eine breite Auswahl an Dufttypen anbieten. Erfindungsgemäße Riechstoffmischungen und parfümierte Produkte enthaltend ein oder mehrere Verbindungen der Formel (I) werden weiter unten beschrieben. Die Mischung enthält 17-Oxacycloheptadec-9-en-1-on, insbesondere (9Z)-17-Oxacycloheptadec-9-en-1-on (Ambrettolide, CAS No. 2864551-4).

Eine erfindunqsqemäße Mischung enthält ein Isomerengemisch aus trans- und cis-konfigurietem 17-Oxacycloheptadec-9-en-1-on und liegt in einer solchen Mischung (jeweils) das Gewichtsverhältnis von der Verbindung (E) zu der Verbindung (Z) (wie hierin beschrieben) im Bereich von 10 : 90 bis 60 : 40.

In diesem Zusammenhang sei exemplarisch auf die folgenden Ergebnisse zur Untersuchung der olfaktorischen Eigenschaften erfindungsgemäßer Mischungen (und Vergleichsmischungen) verwiesen:

Die olfaktorischen Eigenschaften ausgewählter Isomerengemische von Cycllohexadec-8-en-1-on können wie folgt beschrieben werden:
- 67 % trans-Cyclohexadec-8-en-1-on : 31 % cis-Cyclohexadec-8-en-1-on (Referenz): moschusartig, erogen, kosmetisch, balsamisches Geruchsbild und florale Aspekte
- 60 % trans-Cyclohexadec-8-en-1-on : 40 % cis-Cyclohexadec-8-en-1-on (Referenz): moschusartig, erogen, balsamisch, an frische Wald luft erinnernd
   - 40 % trans-Cyclohexadec-8-en-1-on : 60 % cis-Cyclohexadec-8-en-1-on (Referenz): moschusartig, erogen, transparent, an kühle Seeluft erinnernd
   - 20 % trans-Cyclohexadec-8-en-1-on : 80 % cis-Cyclohexadec-8-en-1-on (Referenz): moschusartig, erogen, warm, an trockenes Holz erinnernd.

Die olfaktorischen Eigenschaften ausgewählter Isomerengemische von 17-Oxacycloheptadec-10-en-1-on können wie folgt beschrieben werden:
- 99 % trans-17-Oxacycloheptadec-9-en-1-on : 1 % cis-17-Oxacycloheptadec-9-en-1-on (Vergleichsmischung): moschusartig, erogen, mild ambra-ähnlich, mit floralen Aspekten und geringen fruchtigen Nuancen
- 65 % trans-17-Oxacycloheptadec-9-en-1-on : 30 % cis-17-0xacycloheptadec-9-en-1-on (erfindungsgemäß): moschusartig, erogen, stärker ambra-ähnlich mit mehr fettigen, animalischen Nuancen. Die floralen und fruchtigen Schattierungen treten olfaktiv in den Hintergrund.

Die olfaktorischen Eigenschaften ausgewählter Isomerengemische von Oxacyclohexadecen-2-on können wie folgt beschrieben werden:
- 72 % trans-Oxacyclohexadecen-2-on 22 % cis-Oxacyclohexadecen-2-on (Vergleichsmischung):
   moschusartig, erogen, fettig-ambraähnlich mit einer trocken-holzigen und dezentfruchtigen Ausrichtung
- 65 % trans-Oxacyclohexadecen-2-on 31 % cis-Oxacyclohexadecen-2-on:
   moschusartig, erogen, intensiver talgig-ambraähnlich, mehr an Haut, besonders an gereinigtes Wollfett erinnernd.

Die olfaktorischen Eigenschaften ausgewählter Isomerengemische von Cyclohexadec-5-en-1-on können wie folgt beschrieben werden:
- 37 % trans-Cyclohexadec-5-en-1-on 62 % cis-Cyclohexadec-5-en-1-on (Referenz):
   moschusartig, erogen, mild-ambraähnlich moschusartig, erogen, mild-animalisch mit einem sanft-kosmetischen und transparent-floralem Geruchsbild
- 21 % trans-Cyclohexadec-5-en-1-on 78 % cis-Cyclohexadec-5-en-1-on (Referenz):
   moschusartig, erogen, schwächer animalisch mit einer balsamischen, warmen, an Baum- und Strauchharze erinnernden Duftrichtung.

Vorzugsweise handelt es sich bei einer erfindungsgemäßen Mischung um eine Riechstoffmischung, insbesondere ein Parfümöl. Eine solche Riechstoffmischung enthält vorzugsweise einen oder mehrere zusätzliche Riechstoffe. Besonders bevorzugt ist bzw. sind der zusätzliche bzw. einer, mehrere oder sämtliche der zusätzlichen Riechstoffe ausgewählt aus der Gruppe bestehend aus Alkoholen, Aldehyden, Ketonen, Ethern, Estern und Carboxylaten, vorzugsweise Alkoholen und Aldehyden, insbesondere solchen mit einer Molmasse im Bereich von 150 bis 285 g/mol, vorzugsweise von 210 g/mol oder weniger. Im Zusammenhang mit der vorliegenden Erfindung gilt generell, dass die in einer erfindungsgemäßen Riechstoffmischung gegebenenfalls zusätzlich enthaltenen Riechstoffe eine Molmasse im Bereich von 150 bis 285 g/mol, vorzugsweise von 210 g/mol oder weniger, besitzen. Besonders bevorzugt zu verwendende Riechstoffe werden weiter unten beschrieben.

Im Folgenden werden - beispielhaft anhand exemplarisch untersuchter Isomerengemische von Cyclohexadec-8-en-1-on (wie oben beschrieben) - positive Einflüsse der hierin beschriebenen erfindungsgemäßen Mischungen auf ausgewählte, sogenannte "Messriechstoffe" (molekulare Einzelriechstoffe als Vertreter der chemischfunktionellen Gruppe der Aldehyde und Alkohole) beschrieben:

### Aldehyde:

Dodecanal ist ein aliphatischer Aldehyd mit einem fettig, wachsigen Geruch, der oft auch als typischer "Frischwäscheduft" bezeichnet wird.

Bei einem Zusatz von 10 % (bezogen auf das Gesamtgewicht an Dodecanal) eines Isomerengemisches aus Cyclohexadec-8-en-1-on (hier: 40 % trans-Cyclohexadec-8-en-1-on 60 % cis-Cyclohexadec-8-en-1-on) zu diesem Einzelriechstoff ist überraschenderweise ein sehr positiver Geruch nach Citrusfrüchten, speziell nach Mandarine feststellbar.

2-Methyl Undecanal, auch als iso-Dodencal oder "M.N.A" bekannt, besitzt ein sehr kräftiges Geruchsbild, das mit "trocken", "ambraähnlich" und "krautig" beschrieben wird.

Ein Zusatz von 10% (bezogen auf das Gesamtgewicht an 2-Methyl Undecanal) eines Isomerengemisches aus Cyclohexadec-8-en-1-on (hier: 60 % trans-Cyclohexadec-8-en-1-on : 40 % cis-Cyclohexadec-8-en-1-on) überzeugt mit diffusivem, an Orangenschalen erinnernden Duftcharakter.

Hexylzimtaldehyd, alpha, ist eine iso-cyclische Aldehydform mit einem milden, leicht herbalen und schwach floralem Geruchsbild und erfährt durch den Zusatz von 10 % (bezogen auf das Gesamtgewicht an Hexylzimtaldehyd) eines Isomerengemisches aus Cyclohexadec-8-en-1-on (hier: 40 % trans-Cyclohexadec-8-en-1-on : 60 % cis-Cyclohexadec-8-en-1-on) eine höherwertige, fein-blumige Geruchsentwicklung nach frischen Blüten des Jasminstrauchs.

### Alkohole:

Dimethyloctenol, allgemein bekannt als "Citronellol", zeigt in seiner racemischen Form einen Geruch nach Rosenblüten und Maiglöckchen.

Ergänzt man diesen molekularen Einzelriechstoff mit einem Zehntel seines Anteils durch ein Isomerengemisch aus Cyclohexadec-8-en-1-on (hier: 40 % trans-Cyclohexadec-8-en-1-on : 60 % cis-Cyclohexadec-8-en-1-on), so erhält man wider Erwarten den klaren, frischen, leicht minzigen Geruchseindruck der Laevo-Form des Moleküls, das deutlich nach den frischen Blättern der Duftgeranienstaude riecht.

Dimethyloctadienol, industriell bekannt als "Linalool", mit dem authentischen, blumigholzigen Geruch mit leichtem Citrus-Einfluss, gewinnt durch den Zusatz von 10 % (bezogen auf das Gesamtgewicht an Dimethyloctadienol) eines Isomerengemisches aus Cyclohexadec-8-en-1-on (hier: 60 % trans-Cyclohexadec-8-en-1-on : 40 % cis-Cyclohexadec-8-en-1-on) den elegant-fruchtigen Dufteindruck der Freesienblüte.

Die vorstehend beschriebenen "Messriechstoffe" sind im Rahmen der vorliegenden Erfindung besonders bevorzugt in Kombination mit Verbindungen der Formel (I) (wie hierin beschrieben) in erfindungsgemäßen Riechstoffmischungen einzusetzende Riechstoffe.

Im Zusammenhang mit den hierin beschriebenen erfindungsgemäßen Riechstoffmischungen gilt, dass das Verhältnis der Gesamtmasse an nicht der Formel (I) entsprechenden Riechstoffen zur Gesamtmasse an Verbindung(en) der Formel (I) vorzugsweise größer oder gleich 80 : 20 ist, bevorzugt größer oder gleich 90 : 10, besonders bevorzugt größer oder gleich 95 : 5. Erfindungsgemäß besonders bevorzugte Mengen(-verhältnisse) ergeben sich aus den beigefügten Beispielen.

Erfindungsgemäße Riechstoffmischungen sind üblicherweise bei 25°C und 1013hPa flüssig und in der Regel homogene Lösungen.

Riechstoffmischungen, insbesondere Parfümöle, umfassen häufig synthetische oder natürliche (vorzugsweise) geschmacks- und geruchsneutrale Trägeröle, welche die Duftbeziehungsweise Riechstoffe (als künstliche oder natürliche Stoffe) in hochkonzentrierter Form (sowie gegebenenfalls parfümistische Lösemittel und/oder Hilfsstoffe) enthalten. Entsprechendes gilt für die hierin beschriebenen erfindungsgemäßen Riechstoffmischungen.

Parfümöle (als bevorzugte Ausgestaltung von (erfindungsgemäßen) Riechstoffmischungen) dienen häufig Duftanwendungen. Mit Parfümölen werden beispielsweise Parfüme hergestellt, indem man sie in (z.B. alkoholische) Lösungen gibt, die beim Verdampfen die Duft- beziehungsweise Riechstoffe "mitreißen" und so dem Riechorgan des Anwenders, d.h. des Menschen, den Sinneseindruck eines bestimmten Geruchs vermitteln. Solche Mischungen können beispielsweise ein Parfüm, Eau de Parfüm oder Eau de Toilette sein. Ferner dienen Parfümöle der Erzeugung eines bestimmten Duftes in Wohnräumen, wie beispielsweise bei der Anwendung in Duftlampen, Zerstäubern oder Diffuser. Darüber hinaus können Parfümöle aber auch in unzähligen weiteren Artikeln bzw. Zubereitungen eingesetzt werden, beispielsweise von Schuhcremes bis Haarshampoos, Damenbinden bis WC-Reinigern, Gesichtscremes bis Waschpulver und Katzensteinen.

Beispiele für Riechstoffe, die grundsätzlich vorteilhaft als Bestandteil einer erfindungsgemäßen Riechstoffmischung, insbesondere eines erfindungsgemäßen Parfümöls, verwendet werden können, finden sich z.B. in S. Arctander, Perfume and Flavor Chemicals, Vol. I und II, Montclair, N. J., 1969, Selbstverlag oder H. Surburg, J. Panten, Common Fragrance and Flavor Materials, 5th Ed., Wiley-VCH, Weinheim 2006.

Bevorzugte Etherische Öle, Concretes, Absolues, Resine, Resinoide, Balsame und/oder Tinkturen, die Bestandteil einer erfindungsgemäßen Riechstoffmischung, insbesondere eines erfindungsgemäßen Parfümöls, sein können, sind bevorzugt auszuwählen aus der Gruppe bestehend aus:
Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bitterniandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalrnusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl (engl.: pine oil); Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Teebaum-Öl; Terpentinöl (engl.: turpentine oil); Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl.

Bevorzugte Einzelriechstoffe, die vorzugsweise als Bestandteil einer erfindungsgemäßen Riechstoffmischung, insbesondere eines erfindungsgemäßen Parfümöls, verwendet werden, sind ausgewählt aus der Gruppe
der Kohlenwasserstoffe, dabei bevorzugt 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole, dabei bevorzugt Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale, dabei bevorzugt Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime, dabei bevorzugt 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen, dabei bevorzugt 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile, dabei bevorzugt 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren, dabei bevorzugt (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole, dabei bevorzugt Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone, dabei bevorzugt Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole, dabei bevorzugt Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate; Menthylformiat; Menthylpropionat; Menthylbutyrat; Menthylisobutyrat; Menthylisovalerianat; Menthylhexanoat; Menthylcrotonat; Menthyltiglinat;
der cyclischen Terpenaldehyde und -ketone, dabei bevorzugt Menthon; Isomenthon; 8-Mercaptomenthan-3-on; Carvon; Campher; Fenchon; alpha-Ionon; beta-Ionon; beta-n-Methylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole, dabei bevorzugt 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole, dabei bevorzugt alpha,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether, dabei bevorzugt Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone, dabei bevorzugt 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde, dabei bevorzugt 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone, dabei bevorzugt 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole, dabei bevorzugt 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole, vorzugsweise 1-Cyclohexylethylcrotonat;
der Ester cycloaliphatischer Carbonsäuren, dabei bevorzugt Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole, dabei bevorzugt Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentan-1-ol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren, dabei bevorzugt Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether, dabei bevorzugt 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde, dabei bevorzugt Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone, dabei bevorzugt Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester, dabei bevorzugt Benzoesäure; Phenylessigsäure; Methylbenzoat; Ethylbenzoat; Hexylbenzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen, dabei bevorzugt 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester, dabei bevorzugt Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen, dabei bevorzugt 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone, dabei bevorzugt 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,13-tridecandioat; Cumarin; 2,3-Dihydrocumarin; Octahydrocumarin.

Besonders bevorzugt ist eine erfindungsgemäße Riechstoffmischung, wobei die Menge an erfindungsgemäßer Mischung bzw. die Menge an Verbindung (**E**) und Verbindung (**Z**) (wie hierin beschrieben) ausreicht,
(a) um den bzw. einen oder mehrere unangenehme Geruchseindrücke eines anderen Riechstoffs in der Riechstoffmischung zu maskieren oder zu vermindern,
   und/oder
(b) um den bzw. einen oder mehrere angenehme Geruchseindrücke eines anderen Riechstoffs in der Riechstoffmischung zu verstärken.

Besonders bevorzugt ist es auch, wenn die Gesamtmenge an Verbindungen der Formel (I) bezogen auf das Gesamtgewicht der Riechstoffmischung 1 Gew.-% oder weniger beträgt, vorzugsweise 0,1 Gew.-% oder weniger, besonders bevorzugt 0,001 Gew.-% oder weniger.

Ein weiterer Aspekt der vorliegenden Erfindung betrifft ein parfümiertes Produkt, enthaltend eine erfindungsgemäße Mischung wie hierin beschrieben, vorzugsweise eine erfindungsgemäße Riechstoffmischung, insbesondere ein Parfümöl, in einer sensorisch wirksamen Menge, wobei der Anteil der Mischung bzw. der Riechstoffmischung oder der Verbindungen der Formel (I) bezogen auf das Gesamtgewicht des Produkts vorzugsweise im Bereich von 0,01, bevorzugt 0,1, bis 20, bevorzugt 10 Gew.-%, bevorzugt im Bereich von 0,1 bis 5 Gew.-%, besonders bevorzugt im Bereich von 0,25 bis 3 Gew.-%, liegt.

Für bevorzugt einzusetzende bzw. enthaltene Verbindungen der Formel (I) sowie gegebenenfalls die weiteren Bestandteile einer enthaltenen Riechstoffmischung gilt das oben Gesagte entsprechend.

Klarstellend ist zu erwähnen, dass erfindungsgemäße (parfümierte) Produkte im Rahmen des vorliegenden Textes als absichtlich herbeigeführte bzw. hergestellte Produkte zu verstehen sind, jedoch nicht als natürlich vorkommende Stoffmischungen, wie sie zum Beispiel durch Extraktion von pflanzlichen Ausgangsmaterialien gewonnen werden können.

Bevorzugte Produkte sind beispielsweise Parfüm-Extraits, Eau de Parfums, Eau de Toilettes, Rasierwässer, Eau de Colognes, Pre-shave-Produkte, Splash-Colognes und parfümierte Erfrischungstücher sowie parfümierte oder zu parfümierende saure, alkalische und neutrale Reinigungsmitteln, wie z.B. Fußbodenreiniger, Fensterglasreiniger, Geschirrspülmittel, Bad- und Sanitärreiniger, Scheuermilch, feste und flüssige WC-Reiniger, WC-Sticks, WC-Steine (flüssig oder fest), pulver- und schaumförmige Teppichreiniger, flüssige Waschmittel, pulverförmige Waschmittel, Wäschevorbehandlungsmittel wie Bleichmittel, Einweichmittel und Fleckenentferner, Wäscheweichspüler, Waschseife, Waschtabletten, Desinfektionsmittel, Oberflächendesinfektionsmittel sowie Luftverbesserer in flüssiger, gelartiger oder auf einem festen Träger aufgebrachter Form, besonders zur Desodorierung von Abluft aus Klimaanlagen und industriellen Prozessen, sowie Luftverbesserer in Form von Aerosol- oder Pumpsprays, Wachse und Polituren wie Möbelpolituren, Fußbodenwachse, Schuhcremes, stärkende, imprägnierende oder desodorierende Textilbehandlungsmittel, Windeln, Monatsbinden, Slip-Einlagen, Pflaster, sowie Körperpflegemittel wie z.B. feste und flüssige Seifen, Duschgele, Shampoos, Rasierseife, Rasierschäume, Badeöle, Feuchtreinigungstücher, kosmetische Emulsionen vom Öl-in-Wasser-, vom Wasser-in-Öl- und vom Wasser-in-Öl-in-Wasser-Typ wie z.B. Hautcremes und -lotionen, Gesichtscremes und -lotionen, Sonnenschutzcremes und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukte wie z.B. Haarsprays, Haargele, festigende Haarlotionen, Haarspülungen, permanente und semipermanente Haarfärbemitteln, Haarverformungsmittel wie Kaltwellen- und Haarglättungsmittel, Haarwässer, Haarcremes und -lotionen, Deodorantien und Antiperspirantien wie z.B. Achsel-sprays, Roll-ons, Deosticks, Deocremes, Produkte der dekorativen Kosmetik wie z.B. Lidschatten, Make-ups, Lippenstifte, Mascara sowie Kerzen, Lampenöle, Räucherstäbchen, Tierstreu, Katzenstreu, Insektizide, Repellentien, flüssige und gasförmige Treibstoffe, Heizöle und Heizgase.

Besonders bevorzugt ist ein erfindungsgemäßes Produkt ausgewählt aus der Gruppe bestehend aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und - lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

Einem erfindungsgemäßen Produkt kann auch ein durch eine Verminderung eines unangenehmen Geruchs (insbesondere wie oben beschrieben) oder durch eine Verstärkung eines angenehmen geruchlichen Aspekts (insbesondere wie hierin beschrieben) geruchlich zu verbesserndes Produkt zugrundeliegen.

Gemäß einer bevorzugten Ausgestaltung sind die erfindungsgemäß einzusetzenden Verbindungen der Formel (I) beziehungsweise entsprechende Mischungen davon oder Riechstoffmischungen (wie hierin beschrieben) an einem Trägerstoff adsorbiert, der sowohl für eine feine Verteilung der Verbindungen im Produkt als auch für eine kontrollierte Freisetzung bei der Anwendung sorgt. Derartige Träger können poröse anorganische Materialien wie Kieselgele, Zeolithe, Gipse, Tone, Tongranulate, Gasbeton usw. oder organische Materialien wie Hölzer und Cellulose-basierende Stoffe sein.

Die erfindungsgemäß einzusetzenden Verbindungen der Formel (I) beziehungsweise entsprechende Mischungen davon oder Riechstoffmischungen (wie hierin beschrieben) können auch mikroverkapselt, sprühgetrocknet, als Einschluss-Komplexe oder als Extrusions-Produkte vorliegen und in dieser Form einem Produkt hinzugefügt werden.

Gegebenenfalls können die Eigenschaften solcher derart modifizierten erfindungsgemäß zu verwendenden Verbindungen der Formel (I) beziehungsweise entsprechender Mischungen davon oder Riechstoffmischungen (wie hierin beschrieben) durch sogenanntes "Coaten" mit geeigneten Materialien im Hinblick auf eine gezieltere Freisetzung weiter optimiert werden, wozu vorzugsweise wachsartige Kunststoffe wie z.B. Polyvinylalkohol verwendet werden.

Eine Mikroverkapselung der erfindungsgemäß einzusetzenden Verbindungen der Formel (I) beziehungsweise entsprechender Mischungen davon oder Riechstoffmischungen (wie hierin beschrieben) kann beispielsweise durch das sogenannte Koazervationsverfahren mit Hilfe von Kapselmaterialien, z.B. aus polyurethan-artigen Stoffen oder Weichgelatine, erfolgen. Sprühgetrocknete Verbindungen der Formel (I) können beispielsweise durch Sprühtrocknung einer erfindungsgemäß einzusetzende Substanz, d.h. einer einen Alkohol der Verbindung der Formel (I) oder eine entsprechende Mischung enthaltenden Emulsion bzw. Dispersion hergestellt werden, wobei als Trägerstoff modifizierte Stärken, Proteine, Dextrin und/oder pflanzliche Gummen verwendet werden können. Einschluss-Komplexe können z.B. durch Eintragen von Dispersionen, welche erfindungsgemäß einzusetzende Verbindungen der Formel (I) oder entsprechende Mischungen davon sind beziehungsweise solche umfassen, und Cyclodextrinen oder Harnstoffderivaten in ein geeignetes Lösungsmittel, z.B. Wasser, hergestellt werden. Extrusions-Produkte können durch Verschmelzen der erfindungsgemäß einzusetzenden Verbindung(en) der Formel (I) beziehungsweise entsprechender Mischungen mit einem geeigneten wachsartigen Stoff und durch Extrusion mit nachfolgender Erstarrung, ggf. in einem geeigneten Lösungsmittel, z.B. Isopropanol, erfolgen.

Die erfindungsgemäß einzusetzenden Verbindungen der Formel (I) beziehungsweise entsprechende Mischungen davon oder Riechstoffmischungen (wie hierin beschrieben) können in vielen Zubereitungen bzw. Produkten eingesetzt werden, wobei sie vorzugsweise mit einem oder mehreren der folgenden Hilfs- oder Wirkstoffe kombiniert werden:

Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweißhemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, (Textil-)stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Gemäß einer Ausgestaltung der vorliegenden Erfindung enthält ein bevorzugtes erfindungsgemäßes Produkt, insbesondere ein Deodorant oder dergleichen, (je nach gewünschter Wirkweise) zudem einen oder mehrere der folgenden Wirkstoffe:
(1) Antimikrobiell wirksame Substanzen, die die Entwicklung der für Schweißgeruch verantwortlichen Mikroorganismen hemmen; beispielsweise Triclosan^{®} (5-Chlor-2-(2,4-dichlorphenoxy)phenol), Triclocarban, Chlorhexidin, Chlorhexidinhydrochlorid, Chlorhexidindiacetat, Chlorhexidindigluconat, 2-Phenoxyethanol, Farnesol, Glycerinester und -ether wie Glycerinmonolaurat, Glycerinmonocaprinat, Hexoxyglycerin, Octoxyglycerin (= Ethylhexylglycerin, 3-(2-Ethylhexyloxy-1,2-Propandiol) oder Sensiva^{®} SC 50 (von Schülke & Mayr), aliphatische 1,2-Diole wie z.B. 1,2-Decandiol (EP 1 269 983), araliphatische Alkohole wie beispielsweise beschrieben in EP 799 174, vorzugsweise 4-Methyl-4-phenyl-2-pentanol (Vetikol; WO 03/024907) oder 2-Methyl-4-phenyl-2-butanol (1,1-Dimethyl-3-phenylpropanol, alpha,alpha-Dimethylphenethylcarbinol), I-Menthylmethylether wie beschrieben in WO 02/41861, 2-Benzylheptan-l-ol (Jasmol; 2-n-Pentyl-3-phenylpropan-1-ol), 2,2-Dimethyl-3-phenylpropanol (Muguetalkohol; vgl. US 4,091,090), antimikrobiell wirksame sekundäre Alkohol, wie beispielsweise beschrieben in WO 2005/004601, insbesondere 3-Methyl-6-phenyl-2-hexanol, 4-(2,4-Dimethylphenyl)-2-butanol, 6-(4-Isopropylphenyl)-3-methyl-2-hexanol, 4-(2,4,5-Trimethylphenyl)-2-butanol, 3,3-Dimethyl-4-phenyl-2-butanol, 3-Methyl-4-(2-methylphenyl)-2-butanol, 6-(3,4-Dimethylphenyl)-2-hexanol, aliphatische Carbonsäuren wie 2-Hexyloctansäure, 2-Hexyldecansäure, 2-Butyloctansäure oder 2-Butyldecansäure;
(2) enzyminhibierende Substanzen, die die Wirkung von Enzymen, die an der Bildung von Schweißgeruch beteiligt sind, unterbinden; beispielsweise Citronensäureester und metall-chelatisierende Substanzen wie EDTA (Ethylendiamintetraessigsäure), EGTA [Ethylenbis(oxyethylennitrilo)-tetraessigsäure] und DTPA (Diethylentriaminpentaessigsäure, Pentetic acid);
(3) geruchsabsorbierende Substanzen, die für den Schweißgeruch verantwortliche Stoffe absorbieren; beispielsweise Zinkrizinoleat, Cyclodextrine;
(4) Antiperspirantien, welche die Schweißsekretion hemmen und damit den für den Körpergeruch verantwortlichen Bakterien den Nährboden entziehen. Als Antiperspirantien verwendet man im allgemeinen vorzugsweise adstringierende Metallsalze, besonders anorganische und organische Metallsalze der Elemente Aluminium, Zink, Magnesium, Zinn und Zircon sowie deren Mischungen, wobei insbesonders Halogenide wie Aluminiumchlorid, basische Aluminiumhydroxychloride, Zirconyloxychloride und Zirconylhydroxychloride sowie deren Mischungen verwendet werden. Häufig werden diese Aluminium- und Zirconiumsalze und deren Mischungen auch in einer komplexierten Form verwendet, wobei als Komplexbildner vorzugsweise Propylenglykol, Polyethylenglykol oder Glycin verwendet werden.

Die vorliegende Erfindung betrifft auch ein Verfahren zum Herstellen eines parfümierten Produkts, insbesondere eines erfindungsgemäßen parfümierten Produkts wie hierin beschrieben, umfassend folgende Schritte:
i) Bereitstellen einer erfindungsgemäßen Mischung oder Riechstoffmischung oder einer ersten Verbindung (**E**) und einer zweiten Verbindung (**Z**) wie hierin definiert, in einem Gewichtsverhältnis wie hierin erfindungsgemäß definiert,
ii) Bereitstellen eines oder mehrerer weiterer Bestandteile des herzustellenden parfümierten Produkts (insbesondere solcher wie oben beschrieben), und
iii) Inkontaktbringen oder Mischen der in Schritt ii) bereitgestellten weiteren Bestandteile mit einer sensorisch wirksamen Menge der in Schritt i) bereitgestellten Bestandteile.

Wie sich aus den Ausführungen weiter oben ergibt eignen sich erfindungsgemäße Mischungen, insbesondere solche wie hierin als bevorzugt beschrieben, als Geruchsmodifizierer, vorzugsweise
(a) zum Maskieren oder Vermindern des bzw. eines oder mehrerer unangenehmer Geruchseindrücke eines oder mehrerer unangenehm riechender Stoffe,
   und/oder
(b) zum Verstärken des bzw. eines oder mehrerer angenehmer Geruchseindrücke eines oder mehrerer angenehm riechender Stoffe,
insbesondere in Kombination mit (anderen) Riechstoffen wie weiter oben beschrieben.

Bevorzugt ist hierbei die Verwendung einer erfindungsgemäßen Mischung in einer Zusammensetzung, vorzugsweise einem Parfümöl, die bzw. das einen oder mehrere (weitere) angenehm und/oder unangenehm riechende Stoffe enthält, deren unangenehmer Geruchseindruck durch die erfindungsgemäße Mischung maskiert oder vermindert und/oder deren angenehmer Geruchseindruck durch die erfindungsgemäße Mischung verstärkt wird, wobei diese(r) angenehm und/oder unangenehm riechende Stoff bzw. einer, mehrere oder sämtliche dieser angenehm und/oder unangenehm riechende Stoffe ausgewählt ist bzw. sind aus der Gruppe bestehend aus Alkoholen, Aldehyden, Ketonen, Ethern, Estern und Carboxylaten, vorzugsweise Ketonen und Estern, und/oder eine Molmasse im Bereich von 150 bis 285 g/mol hat bzw. haben.

Hierin beschrieben wird in diesem Zusammenhang auch ein Verfahren
(a) zum Maskieren oder Vermindern des bzw. eines oder mehrerer unangenehmer Geruchseindrücke eines oder mehrerer unangenehm riechender Stoffe,
   und/oder
(b) zum Verstärken des bzw. eines oder mehrerer angenehmer Geruchseindrücke eines oder mehrerer angenehm riechender Stoffe,
umfassend folgenden Schritt:
Vermischen des bzw. der (a) angenehm und/oder (b) unangenehm riechenden Stoffe mit einer erfindungsgemäßen Mischung oder Riechstoffmischung oder, vorzugsweise, einer ersten Verbindung (**E**) und einer zweiten Verbindung (**Z**) wie hierin erfindungsgemäß definiert, in einem Gewichtsverhältnis wie hierin erfindungsgemäß definiert, wobei die Menge an der erfindungsgemäßen Mischung bzw. an Verbindungen (**E**) und (**Z**) ausreicht, um (a) den bzw. die angenehmen Geruchseindrücke des bzw. der angenehm riechenden Stoffe zu verstärken und/oder (b) den bzw. die unangenehmen Geruchseindrücke des bzw. der unangenehm riechenden Stoffe zu vermindern oder zu maskieren.

ilm Rahmen de vorliegenden Textes wird auch ein Verfahren zur Herstellung einer Mischung enthaltend oder bestehend aus einer ersten Verbindung (**E**) und einer zweiten Verbindung (**Z**), wobei die Verbindungen (**E**) und (**Z**) Verbindungen der Formel (**I**) mit identischer Konstitutionsformel sind 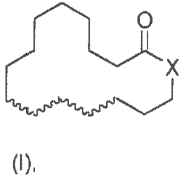
wobei gilt, dass eine der vier gewellten Linien eine Doppelbindung bedeutet und die übrigen gewellten Linien jeweils eine Einfachbindung bedeuten und X ausgewählt ist aus -O-, -CH₂- und -O-CH₂-,
und die erste Verbindung (**E**) trans-konfiguriert und die zweite Verbindung (**Z**) cis-konfiguriert ist, wobei das Gewichtsverhältnis von der Verbindung (**E**) zu der Verbindung (**Z**) in der Mischung im Bereich von 10 : 90 bis 65 : 30, vorzugsweise bis 65 : 35, besonders bevorzugt bis 60 : 40, liegt, wobei die Mischung eine oder mehrere Verbindungen ausgewählt aus der Gruppe bestehend aus Oxacyclohexadecen-2-on, und 17-Oxacycloheptadec-9-en-1-on, insbesondere (9Z)-17-Oxacycloheptadec-9-en-1-on, umfasst, **beschrieben**. Ein solches Verfahren umfasst wenigstens folgende Schritte:
   (i) Bereitstellen einer Ausgangsmischung enthaltend oder bestehend aus einer ersten Verbindung (**E**) und einer zweiten Verbindung (**Z**), wobei die Verbindungen (**E**) und (**Z**) Verbindungen der Formel (I) mit identischer Konstitutionsformel sind 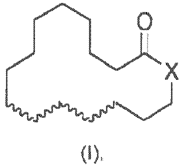
      wobei gilt, dass eine der vier gewellten Linien eine Doppelbindung bedeutet und die übrigen gewellten Linien jeweils eine Einfachbindung bedeuten und X ausgewählt ist aus -O-, -CH₂- und -O-CH₂-,
      und die erste Verbindung (**E**) trans-konfiguriert und die zweite Verbindung (**Z**) cis-konfiguriert ist, wobei das Gewichtsverhältnis von der Verbindung (**E**) zu der Verbindung (**Z**) in der Ausgangsmischung größer als 10 : 90, vorzugsweise größer als 60 : 40, besonders bevorzugt größer als 65 : 35 oder 65 : 30, ist,
   (ii) Durchführen einer säurekatalysierten Isomerisierung, vorzugsweise unter Verwendung von Salpetersäure, der in der Ausgangsmischung enthaltenen Verbindung (**E**), so dass eine Mischung erhalten wird, worin das Gewichtsverhältnis von der Verbindung (**E**) zu der Verbindung (**Z**) in der Mischung im Bereich von 10 : 90 bis 65 : 30, vorzugsweise bis 65 : 35, besonders bevorzugt bis 60 : 40, liegt.

Bevorzugte Ausgestaltungen dieses Verfahrens bzw. bevorzugt einzusetzende Verbindungen ergeben sich aus der vorangehenden Beschreibung.

Eine beispielhafte Verfahrensführung eines solchen Verfahrens ist in Beispiel 3 beschrieben.

Anreicherungen von Z-Isomer lassen sich auch durch eine fraktionierte Destillation erzielen (siehe auch hierzu den Beispielteil unten).

Im Folgenden wird die vorliegende Erfindung anhand ausgewählter Beispiele näher verdeutlicht. Soweit nicht anders angegeben beziehen sich dabei alle Angaben auf das Gewicht.

### Beispiele:

### Beispiel 1: Parfümöl P1 Aldehyde

| **Bestandteil** | **Gew.-Anteil** |
|---|---|
| ALDEHYD C 11 10% in DPG | 16 |
| ALDEHYD C 11 UNDECYLEN 10% in DPG | 18 |
| ALDEHYD C 12 LAURIN 10% in DGP | 14 |
| ALDEHYD C 12 MNA 10% in DGP | 12 |
| HEXENAL TRANS-2 10% in DPG | 1,5 |
| HEXENYLACETAT CIS-3 | 4 |
| VERTOCITRAL | 7,5 |
| MAGNOLAN | 130 |
| MINTONAT | 35 |
| DIHYDROMYRCENOL | 70 |
| ORANGENOEL | 30 |
| NEROLIONE 10% in DPG | 1 |
| HEXYLACETAT | 30 |
| JASMAPRUNAT | 18 |
| ALDEHYD C14 SOG | 50 |
| FRUITATE | 1,8 |
| ETHYLMETHYLBUTYRAT-2 | 8 |
| MANZANATE | 1,2 |
| ALLYLCYCLOHEXYLPROPIONAT | 7 |
| APRIFLOREN | 2 |
| DUPICAL 10% in DPG | 3,5 |
| ETHYLLI NALOOL | 57 |
| DIMETHYLBENZYLCARBINYLBUTYRAT | 6,5 |
| ROSE ABS. | 30 |
| ROSAFEN | 20 |
| DAMASCENON | 1,2 |
| DAMASCON ALPHA | 1,8 |
| BENZYLACETAT | 28 |
| HEDION | 60 |
| HEXYLZIMTALDEHYD ALPHA | 140 |
| PARMANYL. | 3,5 |
| MYSOREACETAT | 35 |
| ISORALDEIN 70 | 28 |
| ISOEUGENOLMETHYLETHER | 3.5 |
| HELIOTROPIN | 3 |
| AGRUMEX LC | 70 |
| AMBROCENIDE 10% in DPG | 0,6 |
| AMBROXIDE | 1,4 |
| | 950 |

Die Komposition ist zu beschreiben als aldehydige, frisch-luftige mit blattgrünen, pfirsichartigen und ambraählichen Dufteindrücken.

Ein Zusatz von 10% (bezogen auf das Gesamtgewicht der in der beurteilten Riechstoffmischung enthaltenen Riechstoffe der Gruppe der Aldehyde) eines Isomerengemisches aus Cyclohexadec-8-en-1-on (hier: 60 % trans-Cyclohexadec-8-en-1-on : 40 % cis-Cyclohexadec-8-en-1-on) resultiert in einer deutlichen, so nicht erwarteten Zunahme der grünen und fruchtigen Dufteindrücke, die an grüne Äpfel erinnern.

### Beispiel 2: Parfümöl P2 Alkohole

| **Bestandteil** | **Gew.-Anteil** |
|---|---|
| ALDEHYD C 12MNA | 5 |
| LINOLAL | 10 |
| VERTOCITRAL | 5 |
| GALBANUM RESIN SYNTH. | 5 |
| MAGNOLAN | 25 |
| MINTONAT | 30 |
| DIHYDROMYRCENOL | 50 |
| ORANGENOEL | 90 |
| PETITGRAINOEL PARAG. | 10 |
| LAVANDIN GROSSO RCO | 30 |
| HERBOXAN | 30 |
| FREESIOL | 30 |
| LINALOOL | 70 |
| GERANIUM RCO | 30 |
| PHENYLETHYLACETAT | 10 |
| PHENYLETHYLALKOHOL | 70 |
| CITRONELLOL | 20 |
| GERANIOL | 20 |
| ROSAFEN | 60 |
| BENZYLACETAT | 50 |
| AMYLZIMTALDEHYD ALPHA | 60 |
| YLANG MC TYPE BASE | 20 |
| AMYLSALICYLAT | 50 |
| PARMANYL | 5 |
| ISORALDEIN 70 | 30 |
| EUGENOL | 10 |
| ANISALDEHYD | 10 |
| HELIOTROPIN | 10 |
| CINNAMYLACETAT | 10 |
| CUMARIN | 10 |
| CYCLABUTE | 10 |
| HERBAFLORAT | 50 |
| PATCHOULIOEL | 10 |
| AMBROXIDE | 5 |
| CITRYLAL | 10 |
| | 950 |

Die Komposition ist zu beschreiben als ein floraler Akkord aus frischen Gartenblumen mit citrischen und an Gartenkräuter erinnernden Duftnoten.

Der Zusatz von 10 % (bezogen auf das Gesamtgewicht der in der beurteilten Riechstoffmischung enthaltenen Riechstoffe der Gruppe der Alkohole) eines Isomerengemisches aus Cyclohexadec-8-en-1-on (hier: 40 % trans-Cyclohexadec-8-en-1-on : 60 % cis-Cyclohexadec-8-en-1-on) lässt die Komposition sehr viel lebendiger, leichter blumig und weniger herbal erscheinen.

### Beispiele 1 und 2 sind Referenzbeispiele.

### Beispiel 3: Herstellung einer erfindungsgemäßen Mischung (Anreicherung von Z-Isomer durch Isomerisierung (z.B. unter Verwendung von Ambrettolide

45g Ambrettolide (17-Oxacycloheptadec-9-en-1-on) (hier: 99% (E) und 0,6% (Z)) werden in 225 ml Isopropanol gelöst und mit 3,0g Salpetersäure (65%ig) versetzt. Anschließend wird unter unter RT 8h gerührt.

Aufarbeitung: Der Ansatz wird mit Soda versetzt, am Rotationsverdampfer eingeengt und mittels KR (Kugelrohrdestillation) destilliert.

In entsprechender Weise kann ausgehend von einem Isomerengemisch von Globalide oder Velvione - anstelle von Ambrettolide - verfahren werden (für Details siehe unten angeführte Tabelle). Globalide und Velvione sind Referenzbeispiele. Isomerisierungsergebnisse:

| **Struktur** | MG | **GC-% Edukt** | | **GC-% Isomerisierung** | |
|---|---|---|---|---|---|
| **Globalide** | | | | | |
| 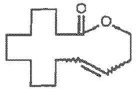 | 238 | 72% E | | 65% E | |
| | | 22% Z | | 31% Z | |

| **Ambrettolide** | | | | | |
|---|---|---|---|---|---|
| 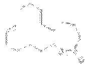 | | 99,0% E | | 67% E | |
| | 252 | 0,6% Z | | 31% Z | |

| **Velvione** | | | | | |
|---|---|---|---|---|---|
| 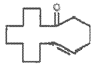 | | | 37% E | | 21% E |
| | | 236 | 62% Z | | 78% Z |

### Beispiel 4: Herstellung einer Mischung (Anreicherung von Z-Isomer durch Destillation (von z.B. Globenone))

### (Referenzbeispiel)

### Apparatur:

4000ml DHK (Dreihalskolben), MGR (Magnetrührer), 1,5m Sulzerkolonne, Pilzheizhaube 2013,2g Globanone werden feindestilliert.

| | ST °C | KT °C | R:D | mbar | Ausbeute in g |
|---|---|---|---|---|---|
| Fraktion 1 bis | 187 | 112,2 | 80:2 | 3,8 | 32,6 |
| Fraktion 2 bis | 191 | 140,7 | 80:2 | 3,0 | 12,8 |
| Fraktion 3 bis | 193 | 141,5 | 80:2 | 3,0 | 31,4 |
| Fraktion 4 bis | 196 | 142,0 | 80:2 | 3,0 | 21,9 |
| Fraktion 5 bis | 197 | 142,3 | 80:2 | 3,0 | 80,3 |
| Fraktion 6 bis | 198 | 141,7 | 100:2 | 2,9 | 26,7 |
| Fraktion 7 bis | 198 | 141,9 | 100:2 | 3,0 | 68,2 |

| | ST °C | KT °C | R:D | mbar | Ausbeute in g |
|---|---|---|---|---|---|
| Fraktion 8 bis | 198 | 142,2 | 100:2 | 3,0 | 25,5 |
| Fraktion 9 bis | 199 | 141,5 | 80:2 | 3,0 | 86,4 |
| Fraktion 10 bis | 200 | 140,4 | 80:2 | 2,7 | 95,1 |
| Fraktion 11 bis | 198 | 140,6 | 80:2 | 2,7 | 84,3 |
| Fraktion 12 bis | 200 | 140,4 | 80:2 | 2,8 | 114,1 |

| Sumpfprobe 1 SP-1 | | | | | |
|---|---|---|---|---|---|
| Fraktion 13 bis | 199 | 141,6 | 80:2 | 2,9 | 249,4 |
| Fraktion 14 bis | 198 | 141,6 | 40:2 | 3,0 | 198,0 |
| Fraktion 15 bis | 198 | 142,5 | 40:2 | 3,1 | 193,2 |

| Sumpfprobe 2 SP-2 | | | | | |
|---|---|---|---|---|---|
| Fraktion 16 bis | 200 | 140,8 | 80:2 | 2,6 | 132,5 |
| Fraktion 17 bis | 203 | 139,7 | 80:2 | 2,6 | 83,6 |
| Fraktion 18 bis | 196 | 140,2 | 80:2 | 2,6 | 9,3 |
| Hold Up | - | - | - | - | 55,7 |
| Rückstand | - | - | - | - | 409 |

Vom Rückstand werden 27,0g KR-destilliert

| 27,0g Eins | T°C | R:D | mbar | Ausbeute in g |
|---|---|---|---|---|
| KRD-Fr. | 150 | 0:1 | 1,4 | 21,4 |
| KRD-Rü. | - | - | - | 5,8 verw. |

### "KRD-2"

| 382g Eins | T °C | R:D | mbar | Ausbeute in g |
|---|---|---|---|---|
| KRD-Fr. | 153 | 0:1 | 0,9 | 303,4 |
| KRD-Rü. | - | - | - | 75,3 |

### KRD-2 wird nochmal destilliert!

Apparatur: 500ml DHK, MGR, 50cm FKK (Füllkörperkolonne) mit Wilsonspiralen, Pilzheizhaube

| *Einsatz KRD-2* | ST °C | KT°C | R:D | mbar | Ausbeute in g |
|---|---|---|---|---|---|
| RF | 176 | 109 | 1:0 | 0,5 | - |
| Fraktion 1 bis | 174 | 122 | 80:2 | 0,8 | 11,5 |
| Fraktion 2 bis | 176 | 125 | 80:2 | 0,9 | 13,6 |
| Fraktion 3 bis | 179 | 127 | 80:2 | 1,0 | 19,9 |
| Fraktion 4 bis | 180 | | 80:2 | 0,9 | 14,9 |
| Fraktion 5 bis | 177 | 130 | 80:2 | 1,1 | 24,0 |

| *Einsatz KRD-2* | ST °C | KT °C | R:D | mbar | Ausbeute in g |
|---|---|---|---|---|---|
| Fraktion 6 bis | 176 | 130 | 80:2 | 1,1 | 16,9 |
| Fraktion 7 bis | 175 | 129 | 80:2 | 1,1 | 13,2 |
| Fraktion 8 bis | 180 | 149 | 80:2 | 3,4 | 22,4 |

| Sumpfprobe SP-1 | | | | | |
|---|---|---|---|---|---|
| Fraktion 9 bis | 184 | 19 | 60:2 | 3,4 | 30,1 |
| Fraktion 10 bis | 182 | 130 | 60:2 | 1,0 | 36,2 |
| Fraktion 11 bis | 194 | 135 | 40:2 | 1,3 | 57,3 |
| Hold Up | - | - | - | - | 10,0 |
| Rückstand | - | - | - | - | 34,2 |

### Analysen:

**GC(FGC811)DB-1:**

| Index | Eins. | Fr.1 | Fr.2 | Fr.3 | Fr.4 | Fr.5 | Fr.6 | Fr.7 | Fr.8 | Fr.9 | Fr.10 | % |
|---|---|---|---|---|---|---|---|---|---|---|---|---|
| *VL* | | *ca. 28* | *3,0* | *3,9* | *1,7* | *0,4* | *0,5* | *0,4* | *0,3* | *0, 2* | *0,3* | *Vorlauf* |
| 1856 | | 4,2 | 4,2 | 3,4 | 3,1 | 2,0 | 1,6 | 1,2 | 1,1 | 0,9 | 0,8 | "7-E" |
| 1883 | 68,0 | 60,3 | 81,7 | 80,8 | 82,4 | 83,7 | 85,1 | 83,5 | 82,9 | 81,9 | 82,0 | trans |
| 1888 | 29,8 | 7,5 | 11,0 | 11,8 | 12,8 | 13,9 | 12,8 | 14,9 | 15,7 | 17,0 | 16,9 | cis |
| Index | Eins. | Fr.11 | Fr.12 | Fr. 13 | Fir.14 | Fr.15 | Fr.16 | Fr.17 | Fr.18 | Rue | HU | % |
| *VL* | | *0,3* | *0,2* | *0,1* | *0,1* | - | - | - | *0,1* | - | *0,1* | *Vorlauf* |
| 1856 | | 0,7 | 0,5 | 0,5 | 0,3 | 0,2 | 0,2 | 0,2 | 0,2 | - | 0,2 | "7-E" |
| 1883 | 68,0 | 81,9 | 80,5 | 79,3 | 76,5 | 71,3 | 63,7 | 60,0 | 59,3 | 30,5 | 51,4 | trans |
| 1888 | 29,8 | 17,1 | 18,8 | 20,2 | 23,2 | 28,5 | 36,1 | 39,8 | 40,4 | 62,9 | 46,3 | cis |

| Index | | Eins. | | | KRD | | | KRD-2 | | | % | |
|---|---|---|---|---|---|---|---|---|---|---|---|---|
| *VL* | | | | | - | | | - | | | *Vorlauf* | |
| 1856 | | | | | - | | | - | | | "7-E" | |
| 1883 | | 68,0 | | | 30,8 | | | 30,4 | | | trans | |
| 1888 | | 29,8 | | | 62,6 | | | 63,2 | | | cis | |
| *NL* | | | | | 6,6 | | | *6,4* | | | *Nachlauf* | |

**KRD-2-Destillation:**

| Index | KRD-2 | Fr.1 | Fr.2 | Fr.3 | Fr.4 | Fr.5 | Fr.6 | Fr.7 | Fr.8 | SP-1 | Fr.9 | Fr.10 | % |
|---|---|---|---|---|---|---|---|---|---|---|---|---|---|
| *VL* | - | *0,1* | - | *0,3* | - | - | - | - | - | - | - | - | *Vorlauf* |
| 1856 | - | - | 0,4 | 0,3 | - | 0,1 | 0,1 | 0,1 | 0,1 | - | 0,1 | 0,1 | "7-E" |
| 1883 | 30,4 | 34,2 | 35,2 | 40,2 | 35,1 | 34,9 | 35,7 | 33,9 | 32,9 | 26,9 | 32,0 | 32,7 | trans |
| 1888 | 63,2 | 63,4 | 62,4 | 54,8 | 63,1 | 63,1 | 62,0 | 63,8 | 64,3 | 62,5 | 65,5 | 62,2 | cis |
| *NL* | *6,4* | *2,3* | *2,0* | *4,4* | 1,8 | 1,9 | *2,2* | *2,2* | *2,7* | *10,6* | 2,4 | *50* | *Nachlauf* |

| Index | KRD-2 | | Fr.11 | Rue | HU % | | | | | | | | |
|---|---|---|---|---|---|---|---|---|---|---|---|---|---|
| *VL* | - | - | - | - | *Vorlauf* | | | | | | | | |
| 1856 | - | 0,2 | 0,1 | 0,4 | "7-E" | | | | | | | | |
| 1883 | 30,4 | 28,6 | 15,4 | 24,7 | trans | | | | | | | | |
| 1888 | 63,2 | 66,4 | 46,0 | 61,5 | cis | | | | | | | | |
| *NL* | 6,4 | 4,8 | 38,5 | *13, δ* | *Nachlauf* | | | | | | | | |

## Patentansprüche

1. Mischung, enthaltend oder bestehend aus einer ersten Verbindung (**E**) und einer zweiten Verbindung (**Z**), wobei die Verbindungen (**E**) und (**Z**) Verbindungen der Formel (I) mit identischer Konstitutionsformel sind 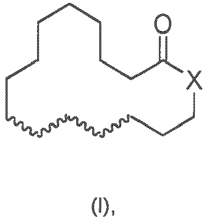
wobei gilt, dass eine der vier gewellten Linien eine Doppelbindung bedeutet und die übrigen gewellten Linien jeweils eine Einfachbindung bedeuten und X ausgewählt ist aus -O-, -CH₂- und -O-CH₂-,
und die erste Verbindung (**E**) trans-konfiguriert und die zweite Verbindung (**Z**) cis-konfiguriert ist, wobei das Gewichtsverhältnis von der Verbindung (**E**) zu der Verbindung (**Z**) in der Mischung im Bereich von 10 : 90 bis 65 : 30, vorzugsweise bis 65 : 35, besonders bevorzugt bis 60 : 40, liegt,
wobei die in der Mischung enthaltene Verbindung der Formel (I) bzw. zumindest eine Verbindung der Formel (I) ausgewählt ist aus der Gruppe bestehend aus 17-Oxacycloheptadec-9-en-1-on.

2. Mischung nach Anspruch 1, wobei die Mischung eine Riechstoffmischung ist, vorzugsweise ein Parfümöl, und zudem vorzugsweise einen oder mehrere zusätzliche Riechstoffe enthält.

3. Mischung nach Anspruch 2, wobei der zusätzliche bzw. einer, mehrere oder sämtliche der zusätzlichen Riechstoffe ausgewählt ist bzw. sind aus der Gruppe bestehend aus Alkoholen, Aldehyden, Ketonen, Ethem, Estern und Carboxylaten, vorzugsweise Aldehyden und Alkoholen.

4. Mischung nach Anspruch 2 oder 3, wobei der zusätzliche bzw. einer, mehrere oder sämtliche der zusätzlichen Riechstoffe eine Molmasse im Bereich von 150 bis 285 g/mol hat bzw. haben, vorzugsweise von 210 g/mol oder weniger.

5. Riechstoffmischung nach einem der Ansprüche 2 bis 4, vorzugsweise Parfümöl, wobei das Verhältnis der Gesamtmasse an nicht der Formel (I) entsprechenden Riechstoffen zur Gesamtmasse an Verbindung(en) der Formel (I) größer oder gleich 80 : 20 ist, bevorzugt größer oder gleich 90 : 10, besonders bevorzugt größer oder gleich 95 : 5.

6. Riechstoffmischung nach einem der Ansprüche 2 bis 5, wobei die Menge an Mischung nach Anspruch 1 bzw. die Menge an Verbindung (**E**) und Verbindung (**Z**) ausreicht,
(a) um den bzw. einen oder mehrere unangenehme Geruchseindrücke eines anderen Riechstoffs in der Riechstoffmischung zu maskieren oder zu vermindern,
und/oder
(b) um den bzw. einen oder mehrere angenehme Geruchseindrücke eines anderen Riechstoffs in der Riechstoffmischung zu verstärken.

7. Riechstoffmischung nach einem der Ansprüche 2 bis 6, vorzugsweise Parfümöl, wobei die Gesamtmenge an Verbindungen der Formel (I) bezogen auf das Gesamtgewicht der Riechstoffmischung 1 Gew.-% oder weniger beträgt, vorzugsweise 0,1 Gew.-% oder weniger, besonders bevorzugt 0,001 Gew.-% oder weniger.

8. Parfümiertes Produkt, enthaltend eine Mischung nach Anspruch 1 oder, vorzugsweise, eine Riechstoffmischung nach einem der Ansprüche 2 bis 7, vorzugsweise ein Parfümöl, in einer sensorisch wirksamen Menge, wobei der Anteil der Mischung bzw. der Riechstoffmischung bezogen auf das Gesamtgewicht des Produkts vorzugsweise im Bereich von 0,01, bevorzugt 0,1, bis 20, bevorzugt 10 Gew.-%, bevorzugt im Bereich von 0,1 bis 5 Gew.-%, besonders bevorzugt im Bereich von 0,25 bis 3 Gew.-%, liegt.

9. Parfümiertes Produkt nach Anspruch 8, wobei das Produkt ausgewählt ist aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und - lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

10. Verfahren zum Herstellen eines parfümierten Produkts, insbesondere eines parfümierten Produkts nach einem der Ansprüche 8 oder 9, umfassend folgende Schritte:
i) Bereitstellen einer Mischung nach Anspruch 1 oder einer Riechstoffmischung nach einem der Ansprüche 2 bis 7 oder einer ersten Verbindung (**E**) und einer zweiten Verbindung (**Z**) wie in Anspruch 1 definiert in einem Gewichtsverhältnis wie in Anspruch 1 definiert,
ii) Bereitstellen eines oder mehrerer weiterer Bestandteile des herzustellenden parfümierten Produkts, und
iii) Inkontaktbringen oder Mischen der in Schritt ii) bereitgestellten weiteren Bestandteile mit einer sensorisch wirksamen Menge der in Schritt i) bereitgestellten Bestandteile.

11. Verwendung einer Mischung nach Anspruch 1 oder einer Riechstoffmischung nach einem der Ansprüche 2 bis 7 als Geruchsmodifizierer, vorzugsweise
(a) zum Maskieren oder Vermindern des bzw. eines oder mehrerer unangenehmer Geruchseindrücke eines oder mehrerer unangenehm riechender Stoffe,
und/oder
(b) zum Verstärken des bzw. eines oder mehrerer angenehmer Geruchseindrücke eines oder mehrerer angenehm riechender Stoffe.

12. Verwendung nach Anspruch 11 in einer Zusammensetzung, vorzugsweise einem Parfümöl, die bzw. das einen oder mehrere (weitere) angenehm und/oder unangenehm riechende Stoffe enthält, deren unangenehmer Geruchseindruck durch die Mischung nach Anspruch 1 maskiert oder vermindert und/oder deren angenehmer Geruchseindruck durch die Mischung nach Anspruch 1 verstärkt wird, wobei diese(r) angenehm und/oder unangenehm riechende Stoff bzw. einer, mehrere oder sämtliche dieser angenehm und/oder unangenehm riechende Stoffe
- ausgewählt ist bzw. sind aus der Gruppe bestehend aus Alkoholen, Aldehyden, Ketonen, Ethem, Estern und Carboxylaten, vorzugsweise Aldehyden und Alkoholen,
und/oder
- eine Molmasse im Bereich von 150 bis 285 g/mol hat bzw. haben.

## Claims

1. Mixture containing or consisting of a first compound (**E**) and a second compound (**Z**), wherein the compounds (**E**) and (**Z**) are compounds of formula (I) with identical constitutional formula 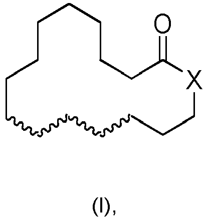
wherein one of the four wavy lines is a double bond and the remaining wavy lines are a single bond, respectively, and X is selected from -O-, -CH₂ - and -O-CHz-,
and the first compound (**E**) is trans-configured and the second compound (**Z**) is cis-configured, wherein the weight ratio of the compound (**E**) to the compound (**Z**) in the mixture is in the range of from 10 : 90 to 65 : 30, preferably to 65 : 35, particularly preferably to 60 : 40,
wherein the compound of formula (I) or at least one compound of formula (I) contained in the mixture is selected from the group consisting of 17-oxacycloheptadec-9-en-1-one.

2. Mixture according to claim 1, wherein the mixture is a fragrance mixture, preferably a perfume oil, and further preferably contains one or more additional fragrances.

3. Mixture according to claim 2, wherein the additional or one, several or all of the additional fragrance(s) is or are selected from the group consisting of alcohols, aldehydes, ketones, ethers, esters and carboxylates, preferably aldehydes and alcohols.

4. Mixture according to claim 2 or 3, wherein the additional or one, several or all of the additional fragrance(s) has or have a molecular weight in the range of from 150 to 285 g/mol, preferably 210 g/mol or less.

5. Fragrance mixture according to any one of the claims 2 to 4, preferably perfume oil, wherein the ratio of the total mass of fragrances not corresponding to formula (I) to the total mass of compound(s) of formula (I) is greater than or equal to 80 : 20, preferably greater than or equal to 90 : 10, particularly preferably greater than or equal to 95 : 5.

6. Fragrance mixture according to any one of the claims 2 to 5, wherein the amount of mixture according to claim 1 or the amount of compound (**E**) and compound (**Z**) is sufficient,
(a) to mask or reduce one or more unpleasant odor impression(s) of another fragrance in the fragrance mixture,
and/or
(b) to enhance one or more pleasant odor impression impression(s) of another fragrance in the fragrance mixture.

7. Fragrance mixture according to any one of the claims 2 to 6, preferably perfume oil, wherein the total amount of compounds of formula (I) relative to the total weight of the fragrance mixture is 1% by weight or less, preferably 0.1% by weight or less, more preferably 0.001% by weight or less.

8. Perfumed product containing a mixture according to claim 1 or, preferably, a fragrance mixture according to any one of the claims 2 to 7, preferably a perfume oil, in a sensory effective amount, wherein the proportion of the mixture or the fragrance mixture relative to the total weight of the product is preferably in the range of from 0.01, preferably 0.1, to 20, preferably 10, % by weight, preferably in the range of from 0.1 to 5 % by weight, particularly preferably in the range of from 0.25 to 3 % by weight.

9. Perfumed product according to claim 8, wherein the product is selected from the group consisting of perfume extracts, eau de parfums, eau de toilettes, shaving lotions, eau de colognes, pre-shave products, splash colognes, perfumed freshening wipes, acid, alkaline and neutral detergents, fabric fresheners, ironing aids, liquid detergents, powder detergents, laundry pretreatment agents, laundry softeners, laundry soaps, laundry tablets, disinfectants, surface disinfectants, air fresheners, aerosol sprays, waxes and polishes, body care products, hand creams and lotions, foot creams and lotions, depilatory creams and lotions, after-shave creams and lotions, tanning creams and lotions, hair care products, deodorants and antiperspirants, decorative cosmetic products, candles, lamp oils, incense sticks, insecticides, repellents, and fuels.

10. Process for preparing a perfumed product, in particular a perfumed product according to any one of the claims 8 or 9, comprising the following steps:
i) Providing a mixture according to claim 1 or a fragrance mixture according to any one of the claims 2 to 7 or a first compound (**E**) and a second compound (**Z**) as defined in claim 1 in a weight ratio as defined in claim 1,
ii) providing one or more further ingredients of the perfumed product to be prepared, and
iii) contacting or mixing the further ingredients provided in step ii) with a sensory effective amount of the ingredients provided in step i).

11. Use of a mixture according to claim 1 or of a fragrance mixture according to any one of the claims 2 to 7 as an odor modifier, preferably
(a) to mask or reduce the or one or several unpleasant odor impression(s) of one or more unpleasantly smelling substances,
and/or
(b) to enhance the or one or several pleasant odor impression(s) of one or more pleasantly smelling substances.

12. Use according to claim 11 in a composition, preferably a perfume oil, which contains one or more (further) pleasantly and/or unpleasantly smelling substances, the unpleasant odor impression of which is masked or reduced by the mixture according to claim 1 and/or the pleasant odor impression of which is enhanced by the mixture according to claim 1, wherein said pleasantly and/or unpleasantly smelling substance or one, several or all of said pleasantly and/or unpleasantly smelling substances
- is/are selected from the group consisting of alcohols, aldehydes, ketones, ethers, esters and carboxylates, preferably aldehydes and alcohols,
and/or
- has/have a molecular weight in the range from 150 to 285 g/mol.

## Revendications

1. Mélange contenant ou se composant d'un premier composé (E) et d'un deuxième composé (Z), les composés (E) et (Z) étant des composés de formule (I) de formule constitutive identique, 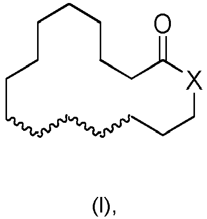
à condition que l'une des quatre lignes ondulées signifie une liaison double et les lignes ondulées restantes signifient chacune une liaison simple et X est choisi parmi -O-, -CH₂- und -O-CHz-,
et le premier composé (E) est de configuration trans et le deuxième composé (Z) est de configuration cis, le rapport pondéral du composé (E) au composé (Z) dans le mélange se situant dans la plage allant de 10 : 90 à 65 : 30, de préférence à 65 : 35, de manière particulièrement préférée à 60 : 40,
dans lequel le composé de formule (I) contenu dans le mélange ou bien au moins un composé de formule (I) est choisi dans le groupe constitué de la 17-oxacyclohep-tadéc-9-én-1-one.

2. Mélange selon la revendication 1, dans lequel le mélange est un mélange de substances odoriférantes, de préférence une huile de parfum, et contient en outre de préférence une ou plusieurs substance(s) odoriférante(s) supplémentaire(s).

3. Mélange selon la revendication 2, dans lequel ladite substance odoriférante supplémentaire ou bien une, plusieurs ou l'ensemble des substances odoriférantes supplémentaires est ou bien sont choisies dans le groupe constitué par les alcools, les aldéhydes, les cétones, les éthers, les esters et les carboxylates, de préférence les aldéhydes et les alcools.

4. Mélange selon la revendication 2 ou 3, dans lequel la substance odoriférante supplémentaire ou bien une, plusieurs ou l'ensemble des substances odoriférantes supplémentaires présente ou bien présentent une masse molaire se situant dans la plage allant de 150 à 285 g/mol, de préférence de 210 g/mol ou moins.

5. Mélange de substances odoriférantes selon l'une quelconque des revendications 2 à 4, de préférence huile de parfum, dans lequel le rapport de la masse totale de substances odoriférantes ne correspondant pas à la formule (I), à la masse totale de composé(s) de formule (I) est supérieur ou égal à 80 : 20, de préférence supérieur ou égal à 90 : 10, de manière particulièrement préférée supérieur ou égal à 95 : 5.

6. Mélange de substances odoriférantes selon l'une quelconque des revendications 2 à 5, dans lequel la quantité de mélange selon la revendication 1 ou bien la quantité de composé (E) et de composé (Z) est suffisante,
(a) pour masquer ou pour réduire la ou bien une ou plusieurs impressions olfactives désagréables d'une autre substance odoriférante dans la composition de substances odoriférantes,
et/ou
(b) pour intensifier la ou bien une ou plusieurs impressions olfactives agréables d'une autre substance odoriférante dans la composition de substances odoriférantes.

7. Mélange de substances odoriférantes selon l'une quelconque des revendications 2 à 6, de préférence huile de parfum, dans lequel la quantité totale de composés de formule (I) par rapport au poids total du mélange de substances odoriférantes est de 1 % en poids ou moins, de préférence de 0,1 % en poids ou moins, de manière particulièrement préférée de 0,001 % en poids ou moins.

8. Produit parfumé contenant un mélange selon la revendication 1 ou, de préférence, un mélange de substances odoriférantes selon l'une quelconque des revendications 2 à 7, de préférence une huile de parfum, en une quantité sensoriellement efficace, dans lequel la proportion du mélange ou bien du mélange de substances odoriférantes par rapport au poids total du produit se situe de préférence dans la plage allant de 0,01, de préférence de 0,1 à 20, de préférence 10 % en poids, de préférence dans la plage allant de 0,1 à 5 % en poids, de manière particulièrement préférée dans la plage allant de 0,25 à 3 % en poids.

9. Produit parfumé selon la revendication 8, dans lequel le produit est choisi dans le groupe constitué par les extraits de parfum, les eaux de parfum, les eaux de toilette, les eaux après-rasage, les Eaux de Cologne, les produits de prérasage, les Splash Cologne, les lingettes rafraîchissantes parfumées, les produits de nettoyage acides, alcalins et neutres, les produits à rafraîchir les textiles, les aides au repassage, les lessives liquides, les lessives en poudre, les produits de pré-traitement de linge, les adoucissants de linge, les savons à laver, les pastilles lave-linge, les désinfectants, les désinfectants de surface, les assainisseurs d'air, les sprays aérosols, les cires et les vernis brillants pour meubles, les produits pour les soins corporels, les crèmes et lotions pour les mains, les crèmes et lotions pour les pieds, les crèmes et lotions dépilatoires, les crèmes et lotions après-rasage, les crèmes et lotions de bronzage, les produits pour les soins capillaires, les déodorants et les antiperspirants, les produits de la cosmétique décorative, les bougies, les huiles de lampe, les bâtonnets d'encens, les insecticides, les répulsifs et les carburants.

10. Procédé de production d'un produit parfumé, en particulier d'un produit parfumé selon l'une quelconque des revendications 8 ou 9, comprenant les étapes suivantes consistant à:
i) fournir un mélange selon la revendication 1 ou un mélange de substances odoriférantes selon l'une quelconque des revendications 2 à 7 ou un premier composé (E) et un deuxième composé (Z) tels que définis dans la revendication 1, dans un rapport pondéral tel que défini dans la revendication 1,
ii) fournir un ou plusieurs autres composants du produit parfumé à produire, et
iii) mettre en contact ou mélanger les autres composants fournis à l'étape ii), avec une quantité sensoriellement efficace des composants fournis à l'étape i).

11. Utilisation d'un mélange selon la revendication 1, ou d'un mélange de substances odoriférantes selon l'une quelconque des revendications 2 à 7, en tant que modificateur d'odeur, de préférence
(a) pour masquer ou pour réduire la ou bien une ou plusieurs impressions olfactives désagréables d'une ou de plusieurs substance(s) ayant une odeur désagréable,
et/ou
(b) pour intensifier la ou bien une ou plusieurs impressions olfactives agréables d'une ou de plusieurs substance(s) ayant une odeur agréable.

12. Utilisation selon la revendication 11 dans une composition, de préférence une huile de parfum, qui contient une ou plusieurs (autres) substances ayant une odeur agréable et/ou désagréable dont l'impression olfactive désagréable est masquée ou réduite par le mélange selon la revendication 1 et/ou dont l'impression olfactive agréable est renforcée par le mélange selon la revendication 1, dans laquelle cette substance ayant une odeur agréable et/ou désagréable ou bien une, plusieurs ou l'ensemble de ces substances ayant une odeur agréable et/ou désagréable
- est ou bien sont choisie(s) dans le groupe constitué par les alcools, les aldéhydes, les cétones, les éthers, les esters et les carboxylates, de préférence les aldéhydes et les alcools,
et/ou
- présente ou bien présentent une masse molaire comprise entre 150 et 285 g/mol.
